(19) Europäisches Patentamt
European Patent Office
Office européen des brevets

(11) EP 1 178 050 B1

(12) EUROPÄISCHE PATENTSCHRIFT

(45) Veröffentlichungstag und Bekanntmachung des Hinweises auf die Patenterteilung:
**23.11.2005 Patentblatt 2005/47**

(51) Int Cl.[7]: **C07F 9/28**, C07C 211/63, C07C 211/14, H01M 10/40

(21) Anmeldenummer: **01115786.4**

(22) Anmeldetag: **11.07.2001**

(54) **Fluoralkylphosphate zur Anwendung in elektrochemischen Zellen**

Fluoroalkylphosphates for use in electrochemical cells

Phosphates de fluoroalkyl pour cellules électrochimiques

(84) Benannte Vertragsstaaten:
**DE FR GB**

(30) Priorität: **04.08.2000 DE 10038858**

(43) Veröffentlichungstag der Anmeldung:
**06.02.2002 Patentblatt 2002/06**

(73) Patentinhaber: **Merck Patent GmbH**
**64293 Darmstadt (DE)**

(72) Erfinder:
- **Heider, Udo, Dr.**
  **64560 Riedstadt (DE)**
- **Schmidt, Michael, Dr.**
  **64342 Seeheim-Jugenheim (DE)**
- **Kühner, Andreas, Dr.**
  **64289 Darmstadt (DE)**
- **Sartori, Peter, Prof. Dr.**
  **86919 Utting (DE)**
- **Ignatyev, Nikolai, Dr.**
  **47058 Duisburg (DE)**

(56) Entgegenhaltungen:
**EP-A- 1 127 888** **WO-A-98/15562**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 178 050 B1

Printed by Jouve, 75001 PARIS (FR)

## Beschreibung

Die vorliegende Erfindung betrifft Fluoralkylphosphate, Verfahren zu ihrer Herstellung sowie ihre Verwendung als Leitsalze in Batterien, Kondensatoren, Superkondensatoren und galvanischen Zellen.

Die Verbreitung von tragbaren elektronischen Geräten, wie z.B. Laptop- und Palmtop-Computern, Mobiltelefonen, oder Videokameras und damit auch der Bedarf nach leichten und leistungsfähigen Batterien hat in den letzten Jahren weltweit dramatisch zugenommen.

Angesichts dieses sprunghaft gestiegenen Bedarfs nach Batterien und den damit verbundenen ökologischen Problemen kommt der Entwicklung von wiederaufladbaren Batterien mit einer langen Lebensdauer eine stetig wachsende Bedeutung zu.

Lithium-Ionen-Batterien und Doppelschichtkondensatoren mit sehr hohen Kapazitäten (sogenannte Super- oder Ultracapacitors) stellen den derzeitigen Stand der Technik dar. In beiden Systemen werden mit $LiPF_6$ bzw. $N(C_2H_5)_4BF_4$ derzeit hydrolyseempfindliche und thermisch instabile Substanzen als Leitsalz verwendet. Im Kontakt mit feuchter Luft bzw. mit Restwasser aus den Lösungsmitteln kann schnell HF entstehen. Neben den toxischen Eigenschaften wirkt HF sehr negativ auf das Zyklenverhalten und somit auf die Performance der elektrochemischen Zellen.

Als Alternative wurden Imide, wie das Bis(trifluormethylsulfonyl)imid oder das Bis(pentafluorethylsulfonyl)imid oder Methanide, wie das Tris(trifluormethylsulfonyl)methanid und deren Derivate vorgestellt. Diese Salze zeigen eine hohe anodische Stabilität und bilden mit organischen aprotischen Lösungsmitteln Lösungen mit hoher Leitfähigkeit aus. Die Imide haben jedoch den Nachteil, daß sie das Aluminiummetall, das als kathodischer Stromableiter in Batterien fungiert, nicht in ausreichendem Maße passiveren. Methanide dagegen lassen sich nur mit einem sehr hohen Aufwand herstellen (Turowsky, Seppelt, Inorg. Chem., 1988, 2135) und reinigen. Zudem hängen die elektrochemischen Eigenschaften wie Oxidationsstabilität und Passivierung von Aluminium sehr stark von der Reinheit des Methanids ab.

Aufgabe der Erfindung war es daher, Leitsalze zur Verfügung zu stellen, die elektrochemisch stabil und einfach herzustellen sind. Eine weitere Aufgabe der Erfindung war es auch, die Lebensdauer und die Leistung von Batterien, Kondensatoren, Superkondensatoren und galvanischen Zellen zu verlängern bzw. zu verbessern.

Überraschenderweise wird diese Aufgabe durch das zur Verfügung stellen von Fluoralkylphosphaten der allgemeinen Formel (1) gelöst,

$$M^+[PF_x(C_yF_{2y+1-z}H_z)_{6-x}]^- \qquad (I)$$

worin
$1 \leq x < 6$
$1 \leq y \leq 8$
$0 \leq z \leq 2y + 1$
und
$M^+$ die Bedeutung
$NR^1R^2R^3R^4$,
$PR^1R^2R^3R^4$,
$P(NR^1R^2)_4$,
$C(NR^1R^2)_3$ besitzt
mit $R^1$ bis $R^4$ H, Alkyl und Aryl ($C_1$-$C_8$) die teilweise durch F, Cl oder Br substituiert sein können.

Bevorzugt werden Fluoralkylphosphate der Formel

$$N(C_2H_5)_4[PF_3(C_2F_5)_3],$$

$$N(CH_3)_4[PF_3(C_4F_9)_3],$$

$$[N(CH_3)_4][PF_3(C_4F_9)_3],$$

$$[N(C_2H_5)_4][PF_3(C_2F_5)_3],$$

$$P[N(CH_3)_2]_4[PF_3(C_2F_5)_3],$$

$$P[N(CH_3)_2]_4[PF_3(C_4F_9)_3],$$

$$[P(CH_3)_4]^+[PF_3(C_2F_5)_3]^-,$$

$$[P(C_2H_5)_4]^+[PF_3(C_2F_5)_3]^-,$$

$$[P(CH_3)_4]^+[PF_3[C_4F_9]_3]^-,$$

$$[P(C_2H_5)_4]^+[PF_3[C_4F_9]_3]^-,$$

$$C[N(CH_3)_2]_3[PF_3(C_2F_5)_3]$$

und

$$C[N(CH_3)_2]_3[PF_3(C_4F_9)_3].$$

Es wurde gefunden, daß die erfindungsgemäßen Verbindungen elektrochemisch sehr stabil sind. Dadurch können die erfindungsgemäßen Fluoralkylphosphate der allgemeinen Formel (I) sowohl in reiner Form als auch in Form ihrer Mischungen als Leitsalze in primären und sekundären Batterien, Kondensatoren, Superkondensatoren und galvanischen Zellen eingesetzt werden. Es ist auch möglich, die erfindungsgemäßen Fluoralkylphosphate gemeinsam mit weiteren, dem Fachmann bekannten Salzen als Leitsalz zu verwenden.

Vorzugsweise werden die erfindungsgemäßen Fluoralkylphosphate in reiner Form als Leitsalz verwendet, da auf diese Weise eine besonders gute Reproduzierbarkeit der elektrochemischen Eigenschaften erzielt werden kann.

Die Herstellung der Fluoralkylphosphate geht von Phosphoranen aus, die nach dem in DE 196 411 38 beschriebenen Verfahren hergestellt werden. Es werden 0,01 bis 4 molare, bevorzugt 0,5 bis 3 molare, besonders bevorzugt 1,5 bis 2,5 molare Lösungen oder Suspensionen dieser Phosphorane oder der Fluoralkylphosphate in organischen aprotischen Lösungsmitteln hergestellt, besonders bevorzugt ausgewählt aus der Gruppe der Carbonate, Ester, Ether, Nitrile, Amide, Ketone, Sulfonsäureester, Sulfonamide, Sulfoxide, Phosphorsäureester, Phosphorane, Chloralkane und deren Mischungen. Bevorzugt werden als Lösungsmittel solche Lösungsmittel oder deren Gemische eingesetzt, die sich direkt zur Anwendung in einer primären oder sekundären Batterie, einem Kondensator, einem Superkondensator oder einer galvanischen Zelle eignen, wie beispielsweise Dimethylcarbonat, Diethylcarbonat, Propylencarbonat, Ethylencarbonat, Ethylmethylcarbonat, Methylpropylcarbonat, 1,2-Dimethoxyethan, 1,2-Diethoxyethan, Methylacetat, γ-Butyrolacton, Ethylacetat, Methylpropionat, Ethylpropionat, Methylbutyrat, Ethylbutyrat, Dimethylsulfoxid, Dioxolan, Sulfolan, Acetonitril, Acrylnitril, Tetrahydrofuran, 2-Methyl-Tetrahydrofuran oder deren Gemische, hergestellt.

Für die Umsetzung mit Fluoralkylphosphaten wird in äquimolaren Mengen, oder im leichten Überschuß, Metall- oder Nichtmetallfluoride, -chloride, -tetrafluroborate oder -hexaflurophosphate, bevorzugt ausgewählt aus der Gruppe $[NR^1R^2R^3R^4]F$, $[NR^1R^2R^3R^4]Cl$, $[PR^1R^2R^3R^4]F$, $[PR^1R^2R^3R^4]Cl$, $[P(NR^1R^2)_4]F$, $[P(NR^1R^2)_4]Cl$, $[C(NR^1R^2)_3]Cl$ und $[C(NR^1R^2)_3]F$, gegebenenfalls deren Formulierungen in Lösungen, zugegeben. Für die Umsetzung mit Phosphoranen wird $N(CH_3)_4F$, $N(C_2H_5)_4F$, $[P[(CH_3)_2]_4]F$ oder $C[N(CH_3)_2]_3F$ in äquimolären Mengen, oder einem leichten Überschuß, zugegeben. Das Gemisch wird im Flüssigkeitsbereich, bevorzugt bei Temperaturen zwischen 0°C und 50°C, besonders bevorzugt bei Raumtemperatur, gerührt. Es wird 0,5 Stunden bis 48 Stunden, bevorzugt 2 bis 12 Stunden, gerührt. Im Fall, daß das erfindungsgemäße Fluoralkylphosphat durch Metathese dargestellt wird, wird das entstehende Nebenprodukt durch Abkühlung des Reaktionsgemisches und anschließender Filtrierung abgetrennt. Bei den Umsetzungen, bei denen kein Nebenprodukt anfällt, kann das Reaktionsgemisch gleich weiter eingesetzt werden.

Die so erhaltenen Elektrolyte eignen sich zum Einsatz in primären Batterien, sekundären Batterien, Kondensatoren, Superkondensatoren und galvanischen Zellen und stellen ebenfalls einen Gegenstand der vorliegenden Erfindung dar.

Die Konzentration der/des erfindungsgemäßen Fluoralkylphosphate(s) in diesen Elektrolyten beträgt vorzugsweise 0,01 bis 4 mol/l, besonders bevorzugt 0,5 bis 3 mol/l.

Gegenstand der Erfindung sind auch primäre Batterien, sekundäre Batterien, Kondensatoren, Superkondensatoren und galvanische Zellen, die wenigstens ein erfindungsgemäßes Fluoralkylphosphat der allgemeinen Formel (1) und ggf. weitere Salze und/oder Zusatzstoffe enthalten. Diese weiteren Salze und Zusatzstoffe sind dem Fachmann z.B. aus Doron Aurbach: Nonaqueous Electrochemistry, Marc Dekker Inc., New York 1999; D.Linden: Handbook of Batterie, Second Edition, McGraw-Hill Inc., New York 1995 und G. Mamantov und A.I. Popov: Chemistry of Nonaqueous Solutions, Current Progress, VCH Verlagsgemeinschaft, Weinheim 1994 bekannt. Sie werden hiermit als Referenz eingeführt und gelten somit als Teil der Offenbarung.

Die erfindungsgemäßen Fluoralkylphosphate können mit gängigen Elektrolyten eingesetzt werden. Geeignet sind z.B. Elektrolyte mit Leitsalzen ausgewählt aus der Gruppe $LiPF_6$, $LiBF_4$, $LiClO_4$, $LiAsF_6$, $LiCF_3SO_3$, $LiN(CF_3SO_2)_2$ oder $LiC(CF_3SO_2)_3$ und deren Mischungen. Die Elektrolyte können auch organische Isocyanate (DE 199 44 603) zur Herabsetzung des Wassergehaltes enthalten. Auch Komplexsalze der allgemeinen Formel (DE 199 51 804) .

$$M^{x+}[EZ]^{y-}_{x/y}$$

worin bedeuten:

x, y 1,2,3,4,5,6
$M^{x+}$ ein Metallion
E eine Lewis-Säure, ausgewählt aus der Gruppe

$BR^1R^2R^3$, $AlR^1R^2R^3$, $PR^1R^2R^3R^4R^5$, $AsR^1R^2R^3R^4R^5$, $VR^1R^2R^3R^4R^5$,
$R^1$ bis $R^5$ gleich oder verschieden, gegebenenfalls durch eine Einfach- oder Doppelbildung direkt miteinander verbunden sind, jeweils einzeln oder gemeinsam die Bedeutung
eines Halogens (F, Cl, Br),
eines Alkyl- oder Alkoxyrestes ($C_1$ bis $C_8$) der teilweise oder vollständig durch F, Cl, Br substituiert sein kann,
eines, gegebenenfalls über Sauerstoff gebundenen aromatischen Rings aus der Gruppe Phenyl, Naphthyl, Anthracenyl oder Phenanthrenyl, der unsubstituiert oder ein- bis sechsfach durch Alkyl ($C_1$ bis $C_8$) oder F, Cl, Br substituiert sein kann
eines, gegebenenfalls über Sauerstoff gebundenen aromatischen heterocyclischen Rings aus der Gruppe Pyridyl, Pyrazyl oder Pyrimidyl, der unsubstituiert oder ein- bis vierfach durch Alkyl ($C_1$ bis $C_8$) oder F, Cl, Br substituiert sein kann, haben können und

Z $OR^6$, $NR^6R^7$, $CR^6R^7R^8$, $OSO_2R^6$, $N(SO_2R^6)(SO_2R^7)$, $C(SO_2R^6)(SO_2R^7)(SO_2R^8)$, $OCOR^6$,

wobei
$R^6$ bis $R^8$ gleich oder verschieden sind, gegebenenfalls durch eine Einfach- oder Doppelbindung direkt miteinander verbunden sind, jeweils einzeln oder gemeinsam die Bedeutung
eines Wasserstoffs oder die Bedeutung wie $R^1$ bis $R^5$ haben, hergestellt durch Umsetzung von einem entsprechenden Bor- oder Phosphor-Lewis-Säure-Solvenz-Adukt mit einem Lithium- oder Tetraalkylammonium-Imid, -Methanid oder -Triflat, können enthalten sein.

Auch Boratsalze (DE 199 59 722) der allgemeinen Formel

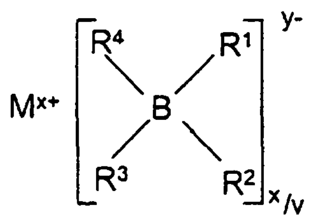

worin bedeuten:

M ein Metallion oder Tetraalkylammoniumion
x,y 1, 2, 3, 4, 5 oder 6

$R^1$ bis $R^4$ gleich oder verschieden, gegebenenfalls durch eine Einfach- oder Doppelbindung direkt miteinander verbundener Alkoxy- oder Carboxyreste ($C_1$-$C_8$) können enthalten sein. Hergestellt werden diese Boratsalze durch Umsetzung von Lithiumtetraalkoholatborat oder einem 1:1 Gemisch aus Lithiumalkoholat mit einem Borsäureester in einem aprotischen Lösungsmittel mit einer geeigneten Hydroxyl- oder Carboxylverbindung im Verhältnis 2:1 oder 4:1.

Auch Additive wie Silanverbindungen der allgemeinen Formel

$$SiR^1R^2R^3R^4$$

mit $R^1$ bis $R^4$ H
$C_yF_{2y+1-z}H_z$
$OC_yF_{2y+1-z}H_z$
$OC(O)C_yF_{2y+1-z}H_z$
$OSO_2C_yF_{2y+1-z}H_z$
und $1 \le x < 6$
$1 \le y \le 8$ und
$0 \le z \le 2y+1$
und
$R^1$-$R^4$ gleich oder verschieden
mit der Bedeutung eines aromatischen Rings aus der Gruppe Phenyl, Naphthyl der unsubstituiert oder ein- oder mehrfach durch F, $C_yF_{2y+1-}{}_zH_z$ oder $OC_yF_{2y+1-z}H_z$, $OC(O)C_yF_{2y+1-z}H_z$, $OSO_2C_yF_{2y+1-z}H_z$, $N(C_nF_{2n+1-}{}_zH_z)_2$ substituiert sein kann, oder
mit der Bedeutung eines heterocyclischen aromatischen Rings aus der Gruppe Pyridyl, Pyrazyl oder Pyrimidyl, die jeweils ein- oder mehrfach mit F, $C_yF_{2y+1-z}H_z$ oder $OC_yF_{2y+1-z}H_z$, $OC(O)C_yF_{2y+1-z}H_z$, $OSO_2C_yF_{2y+1-}{}_zH_z$, $N(C_nF_{2n+1-z}H_z)_2$ substituiert sein können (DE 100 276 26), können enthalten sein.

Die erfindungsgemäßen Verbindungen können auch in Elektrolyte eingesetzt werden, die Lithiumfluoralkylphosphate der folgenden Formel enthalten,

$$Li^+[PF_x(C_yF_{2y+1-z}H_z)_{6-x}]^-$$

worin
$1 \le x \le 5$
$3 \le y \le 8$
$0 \le z \le 2y + 1$
bedeuten und die Liganden $(C_yF_{2y+1-z}H_z)$ gleich oder verschieden sein können, wobei die Verbindungen der allgemeinen Formel,

$$Li^+[PF_a(CH_bF_c(CF_3)_d)_e]^-$$

in der a eine ganze Zahl von 2 bis 5, b = 0 oder 1, c = 0 oder 1, d = 2 und
e eine ganze Zahl von 1 bis 4 bedeuten, mit den Bedingungen, daß b und c nicht gleichzeitig jeweils = 0 bedeuten und die Summe aus a + e gleich 6 ist und die Liganden $(CH_bF_c(CF_3)_d)$ gleich oder verschieden sein können, ausgenommen sind (DE 100 089 55). Das Verfahren zur Herstellung von Lithiumfluoralkylphosphaten ist dadurch gekennzeichnet, daß wenigstens eine Verbindung der allgemeinen Formel

$$H_mP(C_nH_{2n+1})_{3-m},$$

$$OP(C_nH_{2n+1})_3,$$

$$Cl_mP(C_nH_{2n+1})_{3-m},$$

$$F_mP(C_nH_{2n+1})_{3-m},$$

$$Cl_oP(C_nH_{2n+1})_{5-o},$$

$$F_oP(C_nH_{2n+1})_{5-o},$$

in denen jeweils
0 < m < 2, 3 < n < 8 und 0 < o < 4 bedeutet
durch Elektrolyse in Fluorwasserstoff fluoriert wird, das so erhaltene Gemisch der Fluorierungsprodukte durch Extraktion, Phasentrennung und/oder Destillation aufgetrennt wird, und das so erhaltene fluorierte Alkylphosphoran in einem aprotischen Lösungsmittel oder Lösungsmittelgemisch unter Feuchtigkeitsausschluß mit Lithiumfluorid umgesetzt wird, und das so erhaltene Salz nach den üblichen Methoden gereinigt und isoliert wird.

Die erfindungsgemäßen Verbindungen können auch in Elektrolyten eingesetzt werden, die Salze der Formel

$$Li(P(OR^1)_a(OR^2)_b(OR^3)_c(OR^4)_dF_e]$$

worin $0 < a+b+c+d \leq 5$ und $a+b+c+d+e=6$ gilt, und $R^1$ bis $R^4$ unabhängig voneinander Alkyl-, Aryl- oder Heteroarylreste sind, wobei mindestens zwei von $R^1$ bis $R^4$ durch eine Einfach- oder Doppelbindung direkt miteinander verbunden sein können, enthalten (DE 100 16 801). Dargestellt werden die Verbindungen durch Umsetzung von Phosphor (V) -Verbindungen der allgemeinen Formel

$$P(OR^1)_a(OR^2)_b(OR^3)_c(OR^4)_dF_e$$

worin $0 < a+b+c+d \leq 5$ und $a+b+c+d+e=5$ gilt, und $R^1$ bis $R^4$ die oben angegebenen Bedeutungen haben mit Lithiumfluorid in Gegenwart eines organischen Lösungsmittels.

Auch Ionische Flüssigkeiten der allgemeinen Formel

$$K^+A^-$$

worin bedeuten:

$K^+$ ein Kation ausgewählt aus der Gruppe

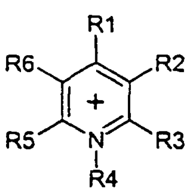

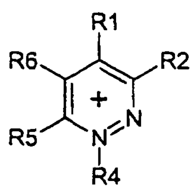

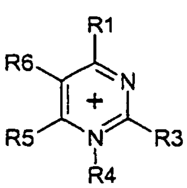

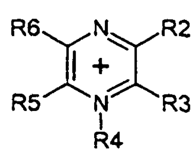

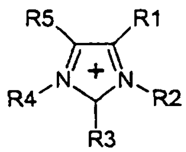
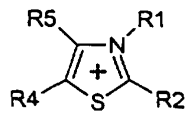
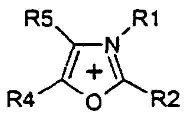
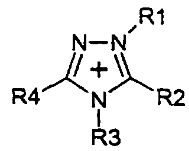

wobei $R^1$ bis $R^5$ gleich oder verschieden, gegebenenfalls durch eine Einfach- oder Doppelbindung direkt miteinander verbunden sind und jeweils einzeln oder gemeinsam folgende Bedeutung haben:

- H,
- Halogen,
- Alkylrest ($C_1$ bis $C_8$) der teilweise oder vollständig durch weitere Gruppen, vorzugsweise F, Cl, $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit 1 <n<6 und 0<x≤13 substituiert sein kann

und

$A^-$ ein Anion ausgewählt aus der Gruppe

$$[B(OR^1)_n(OR^2)_m(OR^3)_o(OR^4)_p]^-$$

mit 0≤n, m, o, p≤4 und
m+n+o+p=4
wobei $R^1$ bis $R^4$ verschieden oder paarweise gleich sind, gegebenenfalls durch eine Einfach- oder Doppelbildung direkt miteinander verbunden sind, jeweils einzeln oder gemeinsam
die Bedeutung eines aromatischen Rings aus der Gruppe Phenyl, Naphthyl, Anthracenyl oder Phenanthrenyl, der unsubstituiert oder ein- oder mehrfach durch $C_nF_{(2n+1-x)}H_x$ mit 1 <n<6 und 0<x≤13 oder Halogen (F, Cl, Br) substituiert sein kann, besitzen,
die Bedeutung eines aromatischen heterocyclischen Rings aus der Gruppe Pyridyl, Pyrazyl oder Pyrimidyl, der unsubstituiert oder ein- oder mehrfach durch $C_nF_{(2n+1-x)}H_x$ mit 1 <n<6 und 0<x≤13 oder Halogen oder Halogen (F, Cl, Br) substituiert sein kann, besitzen,
die Bedeutung eines Alkylrests ($C_1$ bis $C_8$) der teilweise oder vollständig durch weitere Gruppen, vorzugsweise F, Cl,, $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit 1 <n<6 und 0<x≤13 substituiert sein kann, besitzen,
beziehungsweise $OR^1$ bis $OR^4$
einzeln oder gemeinsam die Bedeutung eines aromatischen oder aliphatischen Carboxyl-, Dicarboxyl-, Oxysulfonyl- oder Oxycarboxylrests, der teilweise oder vollständig durch weitere Gruppen, vorzugsweise F, Cl,, $N(C_nF_{(2n+1-x)}H_x)_2$, $O(C_nF_{(2n+1-x)}H_x)$, $SO_2(C_nF_{(2n+1-x)}H_x)$, $C_nF_{(2n+1-x)}H_x$ mit 1 <n<6 und 0<x≤13 substituiert sein kann, besitzen (DE 100 265 65), können im Elektrolyten enthalten sein. Auch Ionische Flüssigkeiten $K^+A^-$ mit $K^+$ definiert wie oben und

$A^-$ ein Anion ausgewählt aus der Gruppe

$$\left[ PF_x(C_yF_{2y+1-z}H_z)_{6-x} \right]^-$$

und $1 \leq x < 6$
$1 \leq y \leq 8$ und
$0 \leq z \leq 2y+1$
können enthalten sein (DE 100 279 95).

Die erfindungsgemäßen Verbindungen können auch in Elektrolyten enthalten sein mit Verbindungen folgender Formel:

$$NR^1R^2R^3$$

worin
$R^1$ und $R^2$ H, $C_yF_{2y+1-z}H_z$ oder $(C_nF_{2n-m}H_m)X$, wobei X ein aromatischer oder heterozyklischer Rest ist, bedeuten und $R^3$ $(C_nF_{2n-m}H_m)Y$, wobei Y ein heterozyklischer Rest ist, oder $(C_oF_{2o-p}H_p)Z$, wobei Z ein aromatischer Rest ist, bedeutet, und wobei n, m, o, p, y und z die folgenden Bedingungen erfüllen: $0 \leq n \leq 6$,
$0 \leq m \leq 2n$,
$2 \leq o \leq 6$,
$0 \leq p \leq 2o$,
$1 \leq y \leq 8$ und
$0 \leq z \leq 2y+1$,
zur Verringerung des Säuregehalts in aprotischen Elektrolytsystemen in elektrochemischen Zellen.

Der Elektrolyt kann auch ein Gemisch enthalten, daß
a) wenigstens ein Lithiumfluoroalkylphosphat-Salz der allgemeinen Formel

$$Li^+ [PF_x(C_yF_{2y+1-z}H_z)_{6-x}]^-$$

worin
$1 \leq x \leq 5$
$1 \leq y \leq 8$ und
$0 \leq z \leq 2y + 1$
bedeuten und die Liganden $(C_yF_{2y+1-z}H_z)$ jeweils gleich oder verschieden sind und
b) wenigstens ein Polymeres (DE 100 58 264) enthält.

Im Elektrolyten können auch Tetrakisfluoroalkylborat-Salze der allgemeinen Formel

$$M^{n+} ([BR_4]^-)_n$$

worin $M^{n+}$ ein einwertiges, zweiwertiges oder dreiwertiges Kation ist, die Liganden R jeweils gleich sind und für $(C_xF_{2x+1})$ mit $1 \leq x \leq 8$ stehen
und n = 1, 2 oder 3 ist (DE 100 558 11) enthalten sein. Das Verfahren zur Herstellung von Tetrakisfluoroalkylborat-Salzen ist dadurch gekennzeichnet, daß wenigstens eine Verbindung der allgemeinen Formel $M^{n+} ([B(CN)_4]^-)_n$ worin $M^{n+}$ und n die oben angegebene Bedeutung haben, durch Umsetzung mit wenigstens einem Fluorierungsmittel in wenigstens einem Lösungsmittel fluoriert und die so erhaltene fluorierte Verbindung nach den üblichen Methoden gereinigt und isoliert wird.

Der Elektrolyt kann auch Boratsalze der allgemeinen Formel

$$M^{n+} [BF_x(C_yF_{2y+1-z}H_z)_{4-x}]_n^-$$

enthalten, worin bedeuten:

| | |
|---|---|
| $1 < x < 3$, | $1 \leq y \leq 8$ und $0 \leq z \leq 2y+1$ und |
| M | ein- bis dreiwertiges Kation ($1 \leq n \leq 3$), außer Kalium und Barium, |

insbesondere:
Li,

$$NR^1R^2R^3R^4,\ PR^5R^6R^7R^8,\ P(NR^5R^6)_kR^7{}_mR^8{}_{4-k-m}$$

(mit k=1-4, m=0-3 und k+m≤4) oder

$$C(NR^5R^6)(NR^7R^8)(NR^9R^{10}),$$

wobei

$R^1$ bis $R^4$ $C_yF_{2y+1-z}H_z$ und
$R^5$ bis $R^{10}$ H oder $C_yF_{2y+1-z}H_z$ oder

ein aromatisches heterozyklisches Kation, insbesondere Stickstoff- und/oder Sauerstoff- und/oder Schwefelhaltige aromatische heterozyklische Kationen (DE 101 031 89). Das Verfahren zur Herstellung dieser Verbindungen ist dadurch gekennzeichnet, daß

a) $BF_3$-Lösungsmittel-Komplexe 1:1 mit Alkyllithium unter Kühlung umgesetzt werden, nach langsamer Erwärmung das Lösungsmittel zu einem Großteil entfernt und anschließend der Feststoff abfiltriert und mit einem geeigneten Lösungsmittel gewaschen wird, oder

b) Lithiumsalze in einem geeigneten Lösungsmittel 1:1 mit $B(CF_3)F_3$-salzen umgesetzt werden, bei erhöhter Temperatur gerührt und nach Entfernen des Lösungsmittels das Reaktionsgemisch mit aprotischen nichtwäßrigen Lösungsmitteln, vorzugsweise mit Lösungsmitteln die in elektrochemischen Zellen verwendet werden, versetzt und getrocknet wird, oder

c) $B(CF_3)F_3$-salze 1:1 bis 1:1,5 mit Lithiumsalzen in Wasser bei erhöhter Temperatur umgesetzt und 0,5 bis 2 Stunden am Siedepunkt erhitzt werden, das Wasser entfernt und das Reaktionsgemisch mit aprotischen nichtwäßrigen Lösungsmitteln, vorzugsweise mit Lösungsmitteln die in elektrochemischen Zellen verwendet werden, versetzt getrocknet wird.

Im Elektrolyten können auch Fluoroalkylphosphat-Salze der allgemeinen Formel

$$M^{n+}\ ([PF_x(C_yF_{2y+1-z}H_z)_{6-x}]^-)_n$$

enthalten sein, worin
$M^{n+}$ ein einwertiges, zweiwertiges oder dreiwertiges Kation ist,
$1 \leq x \leq 5$,
$1 \leq y \leq 8$ und
$0 \leq z \leq 2y + 1$ sind, n = 1, 2 oder 3 bedeuten und die Liganden ($C_yF_{2y+1-z}H_z$) jeweils gleich oder verschieden sind, wobei die Fluoroalkylphosphat-Salze, in denen $M^{n+}$ ein Lithium-Kation ist, sowie die Salze
$M^+([PF_4(CF_3)_2]^-)$ mit $M^+ = Cs^+$, $Ag^+$ oder $K^+$,
$M^+([PF_4(C_2F_5)_2]^-)$ mit $M^+ = Cs^+$,
$M^+([PF_3(C_2F_5)_3]^-)$ mit $M^+ = Cs^+$, $K^+$, $Na^+$ oder para-$Cl(C_6H_4)N_2^+$,
$M^+([PF_3(C_3F_7)_3]^-)$ mit $M^+ = Cs^+$, $K^+$, $Na^+$, para-$Cl(C_6H_4)N_2^+$ oder para-$O_2N(C_6H_4)N_2^+$, ausgenommen sind (DE 100 558 12). Das Verfahren zur Herstellung dieser Fluoroalkylphosphat-Salze ist dadurch gekennzeichnet, daß wenigstens eine Verbindung der allgemeinen Formel

$$H_rP(C_sH_{2s+1})_{3-r},$$

$$OP(C_sH_{2s+1})_3,$$

$$Cl_rP(C_sH_{2s+1})_{3-r},$$

$$F_rP(C_sH_{2s+1})_{3-r},$$

$$Cl_tP(C_sH_{2s+1})_{5-t}$$

und/oder

$$F_tP(C_sH_{2s+1})_{5-t},$$

in denen jeweils
$0 \leq r \leq 2$
$3 \leq s \leq 8$ und
$0 \leq t \leq 4$ bedeuten, durch Elektrolyse in Fluorwasserstoff fluoriert wird, das so erhaltene Gemisch der Fluorierungsprodukte aufgetrennt wird und das so erhaltene fluorierte Alkylphosphoran in einem aprotischen Lösungsmittel oder Lösungsmittelgemisch unter Feuchtigkeitsausschluß mit einer Verbindung der allgemeinen Formel $M^{n+}(F^-)_n$ worin $M^{n+}$ und n die oben angegebene Bedeutung haben, umgesetzt wird, und das so erhaltene Fluoroalkylphosphat-Salz nach den üblichen Methoden gereinigt und isoliert wird.

Die erfindungsgemäßen Verbindungen können in Elektrolyten enthalten sein, die Fluoralkylphosphat-Salze (DE 10109032) der Formel

$$(M^{a+})_b[(C_nF_{2n+1-m}H_m)_yPF_{5-y}(CR_1R_2)_xPF_{5-y}(C_nF_{2n+1-m}H_m)_y]^{(2-)}\ (a*b\ /\ 2)$$

enthalten, worin
$M^{a+}$ ein einwertiges, zweiwertiges oder dreiwertiges Kation,
a = 1, 2 oder 3, b = 2 für a = 1, b = 2 für a = 3, b = 1 für a = 2
und jeweils
$1 \leq n \leq 8$,
$0 \leq m \leq 2$ für n = 1 oder 2,
$0 \leq m \leq 4$ für $3 \leq n \leq 8$,
$1 \leq x \leq 12$,
$0 \leq y \leq 2$, wobei $R_1$ und $R_2$ jeweils identisch oder unterschiedlich sind und ausgewählt sind aus der Gruppe der Fluor-, Wasserstoff-, Alkyl-, Fluoralkyl und Perfluoralkylsubstituenten und,
wobei jeweils die Substituenten $(C_nF_{2n+1-m}H_m)$ identisch oder unterschiedlich sind. Hergestellt werden diese Verbindungen, indem wenigstens ein Fluoro-α,ω-bis(alkylfluorophosphorano)alkan mit wenigstens einem Fluorid-Salz der allgemeinen Formel $(M^{a+})\ [F^-]_a$
worin $(M^{a+})$ und a die oben angegebene Bedeutung hat, in Lösung zu einem Fluoralkylphosphat-Salz umgesetzt und dieses gegebenenfalls nach üblichen Methoden gereinigt und/oder isoliert wird.

Die erfindungsgemäßen Verbindungen können in Elektrolyte für elektrochemische Zellen eingesetzt werden, die Anodenmaterial, bestehend aus beschichteten Metallkernen, ausgewählt aus der Gruppe Sb, Bi, Cd, In, Pb, Ga und Zinn oder deren Legierungen, enthalten (DE 100 16 024). Das Verfahren zur Herstellung dieses Anodenmaterials ist dadurch gekennzeichnet, daß

a) eine Suspension oder ein Sol des Metall- oder Legierungskerns in Urotropin hergestellt wird,

b) die Suspension mit Kohlenwasserstoffen mit $C_5$-$C_{12}$ emulgiert werden,

c) die Emulsion auf die Metall- oder Legierungskerne aufgefällt werden und

d) durch Temperung des Systems die Metallhydroxide bzw. - oxihydroxide in das entsprechende Oxid übergeführt werden.

Die erfindungsgemäßen Verbindungen können auch in Elektrolyte für elektrochemische Zellen eingesetzt werden, mit Kathoden aus gängigen Lithium-Interkalations und Insertionsverbindungen aber auch mit Kathodenmaterialien, die aus Lithium-Mischoxid-Partikel bestehen, die mit einem oder mehreren Metalloxiden (DE 199 22 522) beschichtet sind. Sie können auch aus Lithium-Mischoxid-Partikel bestehen, die mit einem oder mehreren Polymeren

(DE 199 46 066) beschichtet sind. Ebenso können die erfindungsgemäßen Verbindungen in Systemen mit Kathoden eingesetzt werden, die aus Lithium-Mischoxid-Partikeln bestehen, die mit Alkalimetallverbindungen und Metalloxiden ein- oder mehrfach beschichtet sind (DE 100 14 884). Das Verfahren zur Herstellung dieser Materialien ist dadurch gekennzeichnet, daß die Partikel in einem organischen Lösungsmittel suspendiert werden, eine Alkalimetallsalzverbindung suspendiert in einem organischen Lösungsmittel zugegeben wird, Metalloxide gelöst in einem organischen Lösungsmittel zugegeben werden, die Suspension mit einer Hydrolyselösung versetzt wird und anschließend die beschichteten Partikel abfiltriert, getrocknet und calciniert werden. Ebenso können die erfindungsgemäßen Verbindungen in Systemen eingesetzt werden, die Anodenmaterialien mit dotiertem Zinnoxid enthalten (DE 100 257 61). Dieses Anodenmaterial wird hergestellt indem

a) eine Zinnchlorid-Lösung mit Harnstoff versetzt wird,

b) die Lösung mit Urotropin und einer geeigneten Dotierverbindung versetzt wird,

c) das so erhaltene Sol in Petrolether emulgiert wird,

d) das erhaltene Gel gewaschen und das Lösungsmittel abgesaugt sowie

e) das Gel getrocknet und getempert wird.

Ebenso können die erfindungsgemäßen Verbindungen in Systemen eingesetzt werden, die Anodenmaterialien mit reduziertem Zinnoxid enthalten (DE 100 257 62). Dieses Anodenmaterial wird hergestellt indem

a) eine Zinnchlorid-Lösung mit Harnstoff versetzt wird,

b) die Lösung mit Urotropin versetzt wird,

c) das so erhaltene Sol in Petrolether emulgiert wird,

d) das erhaltene Gel gewaschen und das Lösungsmittel abgesaugt wird,

e) das Gel getrocknet und getempert wird und _

f) das erhaltene $SnO_2$ in einem begasbaren Ofen einem reduzierendem Gasstrom ausgesetzt wird.

Die erfindungsgemäßen Fluoralkylphosphate haben den Vorteil, daß sie elektrochemisch stabil sind. Diese Eigenschaft ermöglicht es, Leitsalze mit den erfindungsgemäßen Verbindungen in Batterien, Kondensatoren, Superkondensatoren und galvanische Zellen einzusetzen.

**Beispiele**

Im folgenden wird die Erfindung anhand von Beispielen erläutert. Diese Beispiele dienen lediglich der Erläuterung der Erfindung und schränken den allgemeinen Erfindungsgedanken nicht ein.

Beispiel 1

Darstellung von $N(C_2H_5)_4[PF_3(C_2F_5)_3]$ über $Li[PF_3(C_2F_5)_3]$

Eine 1,5 bis 2,5 molare Lösung von $Li[PF_3(C_2F_5)_3]$ wird mit äquimolaren Mengen an $N(C_2H_5)_4X$ (X=F oder Cl) in Methylenchlorid versetzt. Die Lösung wird mehrere Stunden bei Raumtemperatur gerührt, wobei sich ein leichter Bodensatz von LiX bildet. Danach wird das Reaktionsgemisch weitere 2 Stunden bei -30°C bis -10°C gehalten und der entstandene Niederschlag unter Vakuum bei -30°C bis -10°C abfiltriert. Das Lösungsmittel wird abdestilliert. Nach einer anschließenden Vakuumtrocknung bei 100°C kann das Produkt in Form eines farblosen Granulats erhalten werden.

$^{19}F$-NMR-Spektroskopie ($CD_3CN$; Standard: $CCl_3F$):

-43,6 dm (1F)

-79,7 m (3F)

-81,3 m (6F)

-87,0 dm (2F)

-115,3 m (4F)

-115,7 m (2F)

Beispiel 2

Darstellung von $N(CH_3)_4[PF_3(C_2F_5)_3]$ über $PF_2(C_2F_5)_3$

10 g $PF_2(C_2F_5)_3$ (hergestellt nach DE 198 466 36) werden in Dichlormethan mit einer äquimolaren Menge von $N(CH_3)_4F$ bei -40°C versetzt. Das Gemisch wird mehrere Stunden gerührt und auf Raumtemperatur erwärmt. Das Lösungsmittel wird abdestilliert und $N(CH_3)_4[PF_3(C_2F_5)_3]$ isoliert.

$^{19}F$-NMR-Spektroskopie ($CD_3CN$; Standard: $CCl_3F$):

-44,0 dm (1F)

-80,0 m (3F)

-82 m (6F)

-87,5 dm (2F)

-115,8 m (4F)

-116,2 m (2F)

Beispiel 3

Darstellung von $N(CH_3)_4[PF_3(C_4F_9)_3]$ über $PF_2(C_4F_9)_3$

Die Darstellung erfolgt ausgehend von $PF_2(C_4F_9)_3$ analog dem Beispiel 2. Als Kationenquelle wird $N(CH_3)_4F$ eingesetzt.

Beispiel 4

Darstellung von $P[N(CH_3)_2]_4[PF_3(C_2F_5)_3]$ und $P[N(CH_3)_2]_4[PF_3(C_4F_9)_3]$

Die Darstellung erfolgt analog dem Beispiel 2. Als Kationenquelle wird $P[N(CH_3)_2]_4F$ eingesetzt.

Beispiel 5

Darstellung von $P[N(CH_3)_2]_4[PF_3(C_4F_9)_3]$ und $P[N(CH_3)_2]_4[PF_3(C_2F_5)_3]$

Die Darstellung erfolgt analog dem Beispiel 1. Als Kationenquelle wird $P[N(CH_3)_2]_4Cl$ eingesetzt.

Beispiel 6

Darstellung von $C[N(CH_3)_2]_3[PF_3(C_2F_5)_3]$ und $C[N(CH_3)_2]_3[PF_3(C_4F_9)_3]$

Die Darstellung erfolgt analog dem Beispiel 1. Als Kationenquelle wird $C[N(CH_3)_2]_3Cl$ eingesetzt.

Beispiel 7

Elektrochemische Stabilität der Elektrolyte

In einer Meßzelle mit Edelstahl, Platin- bzw. Goldarbeitselektrode, Lithiumgegenelektrode und Lithiumreferenzelektrode wurden jeweils 5 Zyklovoltammogramme hintereinander aufgenommen. Hierzu wurde ausgehend vom Ruhepotential das Potential zuerst mit einer Vorschubgeschwindigkeit von 10 mV/s bzw. 20 mV/s auf 6 V gegen $Li/Li^+$ erhöht und im weiteren Verlauf zurück auf das Ruhepotential gefahren. Um ein möglichst hohes elektrochemisches Fenster nutzen zu können, wurde eine 1:1 Mischung aus EC und DMC als Lösungsmittel verwendet.

Alle Elektrolyte zeigen hierbei eine anodische Stabilität von über 5V. Abbildung 1 zeigt dies stellvertretend für die $[PF_3(C_2F_5)_3]^-$ enthaltenden Elektrolyte.

## Patentansprüche

**1.** Fluoralkylphosphate der allgemeinen Formel (I)

$$M^+[PF_x(C_yF_{2y+1-z}H_z)_{6-x}]^- \quad (I)$$

worin
$1 \leq x < 6$
$1 \leq y \leq 8$
$0 \leq z \leq 2y + 1$
und
$M^+$ die Bedeutung
$NR^1R^2R^3R^4$,
$PR^1R^2R^3R^4$,
$P(NR^1R^2)_4$,
$C(NR^1R^2)_3$,
besitzt
mit

$R^1$ bis $R^4$ H, Alkyl und Aryl ($C_1$-$C_8$) die teilweise durch F, Cl oder Br substituiert sein können.

**2.** Fluoralkylphosphate gemäß Anspruch 1

a) $N(C_2H_5)_4[PF_3(C_2F_5)_3]$,

b) $N(CH_3)_4[PF_3(C_4F_9)_3]$,

c) $[N(CH_3)_4][PF_3(C_2F_5)_3]$,

d) $[N(C_2H_5)_4][PF_3(C_2F_5)_3]$,

e) $P[N(CH_3)_2]_4[PF_3(C_2F_5)_3]$,

f) $P[N(CH_3)_2]_4[PF_3(C_4F_9)_3]$,

g) $[P(CH_3)_4]^+[PF_3(C_2F_5)_3]^-$,

h) $[P(C_2H_5)_4]^+[PF_3(C_2F_5)_3]^-$,

i) $[P(CH_3)_4]^+[PF_3[C_4F_9]_3]^-$,

k) $[P(C_2H_5)_4]^+[PF_3[C_4F_9]_3]^-$,

l) $C[N(CH_3)_2]_3[PF_3(C_2F_5)_3]$

und

m) $C[N(CH_3)_2]_3[PF_3(C_4F_9)_3]$.

3. Verfahren zur Herstellung von Fluoralkylphosphaten nach Anspruch 1 oder 2, **dadurch gekennzeichnet, daß** Phosphorane mit einem Fluoridsalz oder Fluoralkylphosphate mit einem $M^+$-Salz mit Fluorid-, Chlorid-, Tetrafluorborat-, Hexafluorphosphat- oder anderen Anionen in organischen aprotischen Lösungsmitteln umgesetzt werden.

4. Verfahren nach Anspruch 3, **dadurch gekennzeichnet, daß** Fluoralkylphosphate mit $[NR^1R^2R^3R^4]X$, $[P(NR^1R^2)_4]X$, $[PR^1R^2R^3R^4]X$, $[C(NR^1R^2)_3]X$, mit $X=F^-$, $Cl^-$, $BF_4^-$ oder $PF_6^-$ und $R^{1-4}$ wie in Anspruch 1 definiert, umgesetzt werden.

5. Verfahren nach Anspruch 3, **dadurch gekennzeichnet, daß** Phosphorane mit $N(CH_3)_4F$, $N(C_2H_5)_4F$, $[P[N(CH_3)_2]_4]F$ oder $C[N(CH_3)_2]_3F$ umgesetzt werden.

6. Verfahren nach Anspruch 5, **dadurch gekennzeichnet, daß** die fluorierten Alkylphosphorane in einem Lösungsmittel oder Lösungsmittelgemisch umgesetzt werden, das direkt zur Anwendung in einer primären oder sekundären Batterie, einem Kondensator, einem Superkondensator oder einer galvanischen Zelle geeignet ist.

7. Verfahren nach einem oder mehreren der Ansprüche 3 bis 6, **dadurch gekennzeichnet, daß** organische aprotische Lösungsmittel ausgewählt sind aus der Gruppe der Carbonate, Ester, Ether, Nitrile, Amide, Ketone, Sulfonsäureester, Sulfonamide, Sulfoxide, Phosphorsäureester, Phosphorane, Chloralkane und deren Mischungen.

8. Verfahren nach einem oder mehreren der Ansprüche 3 bis 7, **dadurch gekennzeichnet, daß** als Lösungsmittel Dimethylcarbonat, Diethylcarbonat, Propylencarbonat, Ethylencarbonat, Ethylmethylcarbonat, Methylpropylcarbonat, 1,2-Dimethoxyethan, 1,2-Diethoxyethan, Methylacetat, γ-Butyrolacton, Ethylacetat, Methylpropionat, Ethylpropionat, Methylbutyrat, Ethylbutyrat, Dimethylsulfoxid, Dioxolan, Sulfolan, Acetonitril, Acrylnitril, Tetrahydrofuran, 2-Methyl-Tetrahydrofuran oder deren Mischungen eingesetzt werden.

9. Fluoralkylphosphate erhältlich durch das Verfahren gemäß einem der Ansprüche 3 bis 8.

10. Verwendung wenigstens eines Fluoralkylphosphats gemäß einem der Ansprüche 1 oder 9 als Leitsalz in primären Batterien, sekundären Batterien, Kondensatoren, Superkondensatoren und/oder galvanischen Zellen.

11. Elektrolyte für primäre Batterien, sekundäre Batterien, Kondensatoren, Superkondensatoren und/oder galvanische Zellen enthaltend wenigstens ein Fluoralkylphosphat gemäß einem der Ansprüche 1 oder 9.

12. Elektrolyte gemäß Anspruch 11, **dadurch gekennzeichnet, daß** die Konzentration des/der Fluoralkylphosphate(s) in dem Elektrolyten 0,01 bis 4 mol/l beträgt.

## Claims

1. Fluoroalkyl phosphates of the general formula (I)

$$M^+[PF_x(C_yF_{2y+1-z}H_z)_{6-x}]^- \quad (I)$$

in which
$1 \leq x < 6$
$1 \leq y \leq 8$
$0 \leq z \leq 2y + 1$
and
$M^+$ has the meaning
$NR^1R^2R^3R^4$,
$PR^1R^2R^3R^4$,
$P(NR^1R^2)_4$,
$C(NR^1R^2)_3$, where

$R^1$ to $R^4$ denote H, alkyl or aryl ($C_1$-$C_8$), which may be partially substituted by F, Cl or Br.

**2.** Fluoroalkyl phosphates according to Claim 1

a) $N(C_2H_5)_4[PF_3(C_2F_5)_3]$,

b) $N(CH_3)_4[PF_3(C_4F_9)_3]$,

c) $[N(CH_3)_4][PF_3(C_2F_5)_3]$,

d) $[N(C_2H_5)_4][PF_3(C_2F_5)_3]$,

e) $P[N(CH_3)_2]_4[PF_3(C_2F_5)_3]$,

f) $P[N(CH_3)_2]_4[PF_3(C_4F_9)_3]$,

g) $[P(CH_3)_4]^+[PF_3(C_2F_5)_3]^-$,

h) $[P(C_2H_5)_4]^+[PF_3(C_2F_5)_3]^-$,

i) $[P(CH_3)_4]^+[PF_3[C_4F_9]_3]^-$,

k) $[P(C_2H_5)_4]^+[PF_3[C_4F_9]_3]^-$,

l) $C[N(CH_3)_2]_3[PF_3(C_2F_5)_3]$

and

m) $C[N(CH_3)_2]_3[PF_3(C_4F_9)_3]$.

**3.** Process for the preparation of fluoroalkyl phosphates according to Claim 1 or 2, **characterised in that** phosphoranes are reacted with a fluoride salt or fluoroalkyl phosphates are reacted with an $M^+$ salt with fluoride, chloride,

tetrafluoroborate, hexafluorophosphate or other anions in organic aprotic solvents.

4. Process according to Claim 3, **characterised in that** fluoroalkyl phosphates are reacted with $[NR^1R^2R^3R^4]X$, $[P(NR^1R^2)_4]X$, $[PR^1R^2R^3R^4]X$, $[C(NR^1R^2)_3]X$, where $X=F^-$, $Cl^-$, $BF_4^-$ or $PF_6^-$, and $R^{1-4}$ are as defined in Claim 1.

5. Process according to Claim 3, **characterised in that** phosphoranes are reacted with $N(CH_3)_4F$, $N(C_2H_5)_4F$, $[P[N(CH_3)_2]_4]F$ or $C[N(CH_3)_2]_3F$.

6. Process according to Claim 5, **characterised in that** the fluorinated alkylphos-phoranes are reacted in a solvent or solvent mixture which is directly suitable for use in a primary or secondary battery, a capacitor, a supercapacitor or a galvanic cell.

7. Process according to one or more of Claims 3 to 6, **characterised in that** organic aprotic solvents are selected from the group of the carbonates, esters, ethers, nitriles, amides, ketones, sulfonic acid esters, sulfonamides, sulfoxides, phosphoric acid esters, phosphoranes, chloroalkanes and mixtures thereof.

8. Process according to one or more of Claims 3 to 7, **characterised in that** the solvents employed are dimethyl carbonate, diethyl carbonate, propylene carbonate, ethylene carbonate, ethyl methyl carbonate, methyl propyl carbonate, 1,2-dimethoxyethane, 1,2-diethoxyethane, methyl acetate, γ-butyrolactone, ethyl acetate, methyl propionate, ethyl propionate, methyl butyrate, ethyl butyrate, dimethyl sulfoxide, dioxolane, sulfolane, acetonitrile, acrylonitrile, tetrahydrofuran, 2-methyltetrahydrofuran or mixtures thereof.

9. Fluoroalkyl phosphates obtainable by the process according to one of Claims 3 to 8.

10. Use of at least one fluoroalkyl phosphate according to one of Claims 1 or 9 as conductive salt in primary batteries, secondary batteries, capacitors, super-capacitors and/or galvanic cells.

11. Electrolytes for primary batteries, secondary batteries, capacitors, supercapaci-tors and/or galvanic cells comprising at least one fluoroalkyl phosphate according to one of Claims 1 or 9.

12. Electrolytes according to Claim 11, **characterised in that** the concentration of the fluoroalkyl phosphate(s) in the electrolyte is 0.01 to 4 mol/l.

## Revendications

1. Phosphates de fluoroalkyle de la formule générale (I)

$$M^+[PF_x(C_yF_{2y+1-z}H_z)_{6-x}]^- \qquad (I)$$

dans laquelle
$1 \leq x < 6$
$1 \leq y \leq 8$
$0 \leq z \leq 2y + 1$
et
$M^+$ présente la signification
$NR^1R^2R^3R^4$,
$PR^1R^2R^3R^4$, $P(NR^1R^2)_4$,
$C(NR^1R^2)_3$, où

$R^1$ à $R^4$ représentent H, alkyle ou aryle ($C_1$ - $C_8$), qui peut être partiellement substitué par F, Cl ou Br.

2. Phosphates de fluoroalkyle selon la revendication 1

a) $N(C_2H_5)_4[PF_3(C_2F_5)_3]$,

b) $N(CH_3)_4[PF_3(C_4F_9)_3]$,

c) $[N(CH_3)_4][PF_3(C_2F_5)_3]$,

d) $[N(C_2H_5)_4][PF_3(C_2F_5)_3]$,

e) $P[N(CH_3)_2]_4[PF_3(C_2F_5)_3]$,

f) $P[N(CH_3)_2]_4[PF_3(C_4F_9)_3]$,

g) $[P(CH_3)_4]^+[PF_3(C_2F_5)_3]^-$,

h) $[P(C_2H_5)_4]^+[PF_3(C_2F_5)_3]^-$,

i) $[P(CH_3)_4]^+[PF_3[C_4F_9]_3]^-$,

k) $[P(C_2H_5)_4]^+[PF_3[C_4F_9]_3]^-$,

l) $C[N(CH_3)_2]_3[PF_3(C_2F_5)_3]$

et

m) $C[N(CH_3)_2]_3[PF_3(C_4F_9)_3]$.

**3.** Procédé pour la préparation de phosphates de fluoroalkyle selon la revendication 1 ou 2, **caractérisé en ce que** des phosphoranes sont amenés à réagir avec un sel de fluorure ou des phosphates de fluoroalkyle sont amenés à réagir avec un sel $M^+$ avec des anions de fluorure, de chlorure, de tétrafluoroborate, d'hexafluorophosphate ou d'autres anions dans des solvants aprotiques organiques.

**4.** Procédé selon la revendication 3, **caractérisé en ce que** des phosphates de fluoroalkyle sont amenés à réagir avec $[NR^1R^2R^3R^4]X$, $[P(NR^1R^2)_4]X$, $[PR^1R^2R^3R^4]X$, $[C(NR^1R^2)_3]X$, où $X=F^-$, $Cl^-$, $BF_4^-$ ou $PF_6^-$, et $R^{1-4}$ sont comme défini selon la revendication 1.

**5.** Procédé selon la revendication 3, **caractérisé en ce que** des phosphoranes sont amenés à réagir avec $N(CH_3)_4F$, $N(C_2H_5)_4F$, $[P[N(CH_3)_2]_4]F$ ou $C[N(CH_3)_2]_3F$.

**6.** Procédé selon la revendication 5, **caractérisé en ce que** les alkyles phosphoranes fluorés sont amenés à réagir dans un solvant ou dans un mélange de solvants qui convient directement pour une utilisation dans un accumulateur primaire ou secondaire, un condensateur, un super-condensateur ou une cellule galvanique.

**7.** Procédé selon une ou plusieurs des revendications 3 à 6, **caractérisé en ce que** les solvants aprotiques organiques sont choisis parmi le groupe des carbonates, esters, éthers, nitriles, amides, cétones, esters d'acide sulfonique, sulfonamides, sulfoxydes, esters d'acide phosphorique, phosphoranes, chloroalkanes et des mélanges afférents.

**8.** Procédé selon une ou plusieurs des revendications 3 à 7, **caractérisé en ce que** les solvants utilisés sont carbonate de diméthyle, carbonate de diéthyle, carbonate de propylène, carbonate d'éthylène, carbonate d'éthyle méthyle, carbonate de méthyle propyle, 1,2-diméthoxyéthane, 1,2-diéthoxyéthane, acétate de méthyle, γ-butyrolactone, acétate d'éthyle, propionate de méthyle, propionate d'éthyle, butyrate de méthyle, butyrate d'éthyle, sulfoxyde de diméthyle, dioxolane, sulfolane, acétonitrile, acrylonitrile, tétrahydrofurane, 2-méthyltétra-hydrofurané ou des mélanges afférents.

**9.** Phosphates de fluoroalkyle pouvant être obtenus au moyen du procédé selon l'une des revendications 3 à 8.

**10.** Utilisation d'au moins un phosphate de fluoroalkyle selon l'une des revendications 1 ou 9 en tant que sel conducteur dans des accumulateurs primaires, des accumulateurs secondaires, des condensateurs, des super-condensateurs et/ou des cellules galvaniques.

**11.** Electrolytes pour des accumulateurs primaires, des accumulateurs secondaires, des condensateurs, des super-condensateurs et/ou des cellules galvaniques comprenant au moins un phosphate de fluoroalkyle selon l'une des revendications 1 ou 9.

**12.** Electrolytes selon la revendication 11, **caractérisés en ce que** la concentration en phosphate(s) de fluoroalkyle dans l'électrolyte est de 0,01 à 4 mol/l.